# EUROPÄISCHE PATENTANMELDUNG

(11) **EP 1 949 884 A1**
(43) Veröffentlichungstag der Anmeldung: **30.07.2008**
(21) Anmeldenummer: 07001528.4
(22) Anmeldetag: 24.01.2007
(51) Int. Cl.: A61K 6/083

(54) **Dentalmaterial**

(71) Anmelder: Ernst Mühlbauer GmbH & Co.KG, 25870 Norderfriedrichskoog (DE)
(72) Erfinder: Neffgen, Stephan. Dr, 22459 Hamburg (DE); Ziegler, Silke. Dr, 22529 Hamburg (DE)
(74) Vertreter: Glawe, Delfs, Moll

(57) **Zusammenfassung**

Gegenstand der Erfindung ist ein durch Polymerisation aushärtbares Dentalmaterial, insbesondere ein Fissurenversiegler. Erfindungsgemäß ist vorgesehen, dass darin ein phosphoreszierender Stoff und ein fluoridfreisetzendes Dentalglas enthalten sind.

## Beschreibung

Die Erfindung betrifft ein durch Polymerisation aushärtbares Dentalmaterial, insbesondere einen Fissurenversiegler.

Auf den Kauflächen der Molaren und Prämolaren (Backenzähnen) befindet sich ein natürliches Furchensystem, das Fissuren genannt wird. Die Fissuren sind durch übliche Zahnhygiene nur unzureichend oder gar nicht zu reinigen und daher für Kariesbefall anfällig. Mit einen sehr dünn fließenden Zahnfüllungskunststoff (Fissurenversiegler), können diese natürlichen Furchen dauerhaft aufgefüllt werden, so dass die nun geglätteten Flächen leichter zu reinigen sind. Eine Fissurenversiegelung ist unmittelbar nach Durchbruch des Zahnes am sinnvollsten, da die zu versiegelnde Fissur vor der Versiegelung kariesfrei sein sollte. Mit anderen Worten, bei der Fissurenversiegelung handelt es sich um eine kariesvorsorgende Maßnahme für gesunde Zähne, die bereits im Kindesalter durchgeführt werden sollte, da die Mundhygiene, nicht zuletzt durch die noch nicht ausgeprägte Feinmotorik bei kleinen Kindern, hier ohnehin erschwerter ist. Eine weitere kariesvorsorgende Maßnahme für gesunde Zähne ist die regelmäßige Zufuhr von Fluoridionen, die zur Härtung und Erhaltung des Zahnschmelzes beitragen.

Beispielsweise aus US 6,573,312 B2 ist es bekannt, die Fissuren mit einem polymerisierbaren Fissurenversiegler gegen das Eindringen von Bakterien und damit Kariesbefall zu schützen.

Da wie der Name schon vermuten lässt durch einen Fissurenversiegler große Teile der Zahnoberfläche versiegelt werden, sind fluorhaltigen Hygieneprodukten wie Zahnpasta, Mundspülungen oder auch Fluoridierungsgelen oder -lacken die versiegelten Fissurflächen nicht direkt zugänglich. Um so wichtiger ist es, dass der Fissurenversiegler selbst Fluoridionen an den Zahnschmelz abgibt u.a. um so die Bildung von den Zahnschmelz stärkendem Fluorapatit zumindest zu unterstützen. Dies wird bei Compositen wie etwa bei aus der US 6,573,312 B2 bekannten Fissurenversieglern durch die Zugabe von löslichen Fluoriden, vorzugsweise Natriumfluorid oder fluorhaltigen Harzen (Ionenaustauscher) erreicht *(*Morphis, T.L.; Toumba, K.J.; Lygidakis, N.A.; Int.J.; International Journal of Paediatric Dentistry 2000; 10: 90-98). Diese löslichen Fluoridsalze haben jedoch den Nachteil, dass sie die Fluridionen relativ rasch abgeben und die fluorhaltigen Harze, dass die Gesamtkonzentration an abgegeben Fluoridionen sehr gering ist. Zudem wird die Härte des Fissurenversieglers herabgesetz, wenn sich das Fluoridsalz auflöst. Die erwünschte langanhaltend hohe Fluoridabgabe erreichte man bisher nur bei Verwendung von ionenabgebenden Fluoraluminiumsilikatgläsern in organische Säuren enthaltenden Glasionomerzementen oder sogenannten Compomeren, die säuregruppenhaltige Monomere enthalten und eine Materialklasse darstellen, die Eigenschaften der Composite wie Glasionomerzemente vereint.

Aus US 2006/0194172 A1 ist bekannt, dem polymerisierbaren Dentalmaterial für einen geschädigten Zahn, insbesondere Füllungen und Dentalklebstoffen, ein phosphoreszierendes Material hinzuzufügen um bei erforderlichen späteren Arbeiten am Zahn das üblicherweise in Zahnfarbe ausgewählte Füllungsmaterial bzw. den durch den Dentalklebstoff vermittelten Übergang zum Zahnmaterial unterscheiden zu können. Dadurch kann bei späteren Behandlungen des behandelten Zahn, beispielsweise bei dem Ersatz der Füllung wie sie nach bakterieller Infiltration und Karies am gefüllten Zahn notwendig ist, ein großer Abtrag der Zahnsubstanz vermieden werden.

Anders als bei dem Verlust oder der Beschädigung der Zahnersatzmaterialien wie sie bei geschädigten Zähnen Verwendung finden (Inlays, Füllungen etc.) ist der Verlust der prophylaktischen Versiegelung schmerzfrei. Daher ist eine Routinekontrolle der Versiegelung seitens des Patienten oder Zahnarztes in bestimmten Zeitintervallen erforderlich um einen Versiegelungsverlust rechtzeitig zu erkennen und die Versiegelung zu erneuern.

Der Erfindung liegt die Aufgabe zugrunde, einen ästhetisch ansprechenden Fissurenversiegler zu schaffen, der dem Zahnarzt und dem Patienten selbst eine problemlose Überprüfung des Versiegelungserfolgs ermöglicht.

Die Erfindung löst diese Aufgabe dadurch, dass das Dentalmaterial, insbesondere der Fissurenversiegler, einen phosphoreszierenden Stoff und fluoridfreisetzendes Dentalglas enthält.

Zunächst seien einige im Rahmen der Erfindung verwendete Begriffe erläutert.

Der Begriff "Fissurenversiegler" umfasst jeglichen dünn fließenden Zahnfüllungskunststoff der zur Auffüllung der natürlichen Furchen, Rillen und Grübchen an den Zähnen geeignet ist. Solche Furchen, Rillen und Grübchen finden sich insbesondere an den "höckerigen" Prämolaren und Molaren. Natürliche Furchen, Rillen und Grübchen sind Furchen, Rillen und Grübchen deren Anatomie genetisch vorgegeben ist, d.h. nicht auf Umwelteinflüssen wie beispielsweise Kariesschädigungen beruht. Die Versiegelung erfolgt vorzugsweise solange die zu versiegelnden Zähne (Milchzähne oder bleibende Zähne) noch ungeschädigt, insbesondere kariesfrei sind. Ein geeigneter Zeitpunkt ist das Kindes- bzw. Jugendalter nach dem Durchbruch der Zähne.

Der Begriff "durch Polymerisation aushärtbares Dentalmaterial" umfasst jegliche in der Zahnheilkunde verwendbaren Materialien, bei denen Monomere, Oligomere und/oder Präpolymere durch Polymerisation aushärten können. Insbesondere umfasst dieser Begriff Fissurenversiegler sowie dentale Füllungs- und Restaurationsmaterialien, Stumpfaufbaumaterialien, Befestigungsmaterialien, provisorische und permanente Kronen- und Brückenmaterialien, zahntechnische Werkstoffe zur Herstellung von Inlays, Onlays, Verblendschalen, künstlichen Zähnen und ggf. Modellmaterialien. Besonders bevorzugt umfasst der Begriff Versiegelungsmaterialien, insbesondere oben genannte Fissurenversiegler, Füllungsmaterialien, permanentes Kronen- und Brückenmaterial, sowie zahntechnische Werkstoffe zur Herstellung von Inlays und Onlays.

Ein phosphoreszierender Stoff im Sinne der Erfindung ist jeglicher Stoff, der angeregt durch sichtbares Licht (ggf. sichtbares Licht mit einem hohen Blauanteil) oder UV-Licht eine Lumineszenz mit einer langen Abklingzeitkonstanten (vorzugsweise < 10⁻³s) zeigt. Verschiedene phosphoreszierende Stoffe sind dem Fachmann geläufig und beispielsweise ausführlich beschrieben in Ullmanns Encyclopaedia of Industrial Chemistry, 6. Auflage, Band 20, Seite 273 ff. (luminescent materials). Der phosphoreszierende Stoff kann Sulfide von Erdalkalimetallen und/oder insbesondere Zinksulfide umfassen. Solche phosphoreszierenden Sulfide sind üblicherweise dotiert beispielsweise mit Kupfer, Lanthaniden oder anderen Metallen.

Der phosphoreszierende Stoff kann Erdalkalioxidaluminate umfassen. Beispielsweise sind dotierte Strontiumoxidaluminate kommerziell erhältlich unter dem Handelsnamen Super Luminova^{®}. Solche phosphoreszierenden Materialien sind detailliert beschrieben beispielsweise in EP 0 710 709 B1 oder US 2006/0194172. Die phosphoreszierenden Stoffe zeigen nach Anregung mit sichtbarem Licht in der Dunkelheit ein langes Nachleuchten, das sich über mehrere Stunden erstrecken kann.

Unter fluoridfreisetzenden Dentalgläsern sind sämtliche ionenfreisetzenden Gläser zu verstehen, die in Dentalmaterialien zu verwenden sind. Insbesondere Fluoraluminosilikatgläser wie sie beispielsweise in Dentalzementen, insbesondere in Glasionomerzementen Verwendung finden. Grundbestandteile solcher auch Ionomergläser genannten Dentalgläser sind Siliziumoxid, Aluminiumoxid sowie Oxide bzw. Salze der Erdalkalimetalle. Besonders bevorzugt sind strontiumhaltige Dentalgläser. Zusammensetzungen und Eigenschaften von Ionomergläsern werden beispielsweise in Wilson A.D., McLean J.W.; Glasionomerzemente; Quintessenz Verlags-GmbH 1988 beschrieben.

Die Erfindung hat erkannt, dass die vorgenannten phosphoreszierenden Stoffe für die Verwendung in Fissurenversieglern aufgrund der speziellen Anforderungen an Fissurenversiegler problematisch sind, wie nachfolgend im Einzelnen erläutert wird.

Bei der Fissurenversieglung wird der zu versiegelnde Zahn zunächst gereinigt, trocken gelegt und anschließend mit einer Säure aufgeraut. Durch das Einwirken der notwendigen Säure wird die Schmelzstruktur des Zahnes so verändert, dass eine adäquate Haftbarkeit für den im nächsten Schritt aufgetragenen Versiegler entsteht. Dem Fachmann ist bekannt, dass die eingangs genannten phosphoreszierenden Sulfid dotierten Stoffe nicht säurestabil sind. Er würde diese phosphoreszierenden Stoffe daher gerade nicht als Bestandteil eines Fissurenversieglers, der in einem sauren Milieu aufgetragen wird, in Betracht ziehen. Abgesehen davon enthalten im Stand der Technik verwendeten Fissurenversiegler häufig selbst Säure (z.B. Polyacrylsäure), nämlich dann wenn eine hohe langanhaltende Fluoridfreisetzung realisiert werden soll Problematisch ist ebenfalls, dass unter Umständen später an unversiegelten Bereichen auftretende Kariesherde, die von säurebildenden Kariesbakterien verursacht werden, ebenfalls ein saures Milieu in unmittelbarer Nähe zum Versiegelungsmaterial schaffen können.

Auch die phosphoreszierenden Pigmente auf Erdalkalioxidaluminat-Basis, beispielsweise die eingangs genannten Luminova^{®} Kristalle, würde der Fachmann für die Verwendung in Fissurenversieglern nicht in Betracht ziehen. Problematisch an dem Luminova^{®}-Produkt, und typisch für alle phosphoreszierenden Pigmente auf Erdalkalioxidaluminat-Basis, ist, dass bei längerem direktem Kontakt mit Wasser oder hoher Luftfeuchtigkeit eine Zersetzung mit Bildung einer weislichen Schicht (Hydroxidschicht) an der Oberfläche stattfindet. Damit reduziert sich auch die Leuchtintensität der Kristalle. Mit anderen Worten, die fehlende Hydrolysestabilität beeinträchtigt die Phosphoreszenz in wässriger Umgebung, wie man sie beispielsweise in der Mundhöhle findet, in erheblichen Maße, insbesondere wenn die Funktionsweise der Leuchtkristalle über einen langen Zeitraum sicher gestellt werden soll.

Die Erfindung beruht auf der überraschenden Erkenntnis, das die vorgenannten Nachteile der phosphoreszierenden Stoffe, die einer potentiellen Verwendung in Fissurenversieglern entgegenstehen, vermieden bzw. deutlich verringert werden können, wenn das erfindungsgemäße polymerisierbare Material des Fissurenversieglers neben dem phosphoreszierenden Stoff fluoridfreisetzendes Dentalglas enthält. Gemäß einer bevorzugten Ausführungsform der Erfindung enthält das Dentalglas 10-50 Gew.-% F, 1-10 Gew.-% Na₂O, 10-50 Gew.-% Al₂O₃, 10-50 Gew.-% SiO₂ 1-10 Gew.-% P₂O₅, 1-10 Gew.-% ZnO und 10-50 Gew.-% SrO.

Die Verwendung von fluoridfreisetzendem Dentalglas hat den weiteren Vorteil, dass die den Zahnschmelz härtenden (und damit kariesvorbeugenden) Fluoride permanent und über einen längeren Zeitraum abgegeben werden. Dieses "Fluoriddepot" ist eine wirksame Ergänzung zu den nicht permanenten/sporadischen Fluoridgaben, wie sie beispielsweise durch fluoridhaltige Zahnpasten oder Gele, fluoridiertes Kochsalz oder Trinkwasser erfolgen können.

Besonders vorteilhaft ist es, wenn dass das Dentalglas und/oder der phosphoreszierende Stoff eine durchschnittliche Teilchengröße kleiner 10 *µ*m, bevorzugt kleiner 5 *µ*m, weiter bevorzugt kleiner 3 *µ*m, weiter bevorzugt kleiner 2 *µ*m, weiter bevorzugt kleiner 1 *µ*m, besonders bevorzugt kleiner 0,5 *µ*m aufweist. Gemäß einer bevorzugten Ausführungsform der Erfindung beträgt die maximale Teilchengröße des Dentalglases und/oder der phosphoreszierenden Stoffes kleiner 40 *µ*m, weiter bevorzugt kleiner 30 *µ*m, weiter bevorzugt kleiner 20 *µ*m, weiter bevorzugt kleiner 10 *µ*m, weiter bevorzugt kleiner 5 *µ*m, weiter bevorzugt kleiner 4 *µ*m, weiter bevorzugt kleiner 3 *µ*m, besonders bevorzugt kleiner 2 *µ*m.

Die Erfindung hat erkannt, dass durch die Auswahl entsprechend kleiner Partikelteilchen zum einem die Fließfähigkeit des Versiegelungsmaterials bis zum Boden auch kleinster und schmalster Furchen, Rillen und Grübchen sichergestellt ist und zum anderen keine größeren Teilchen aus der Fissurenversiegelung herausragen bzw. überstehen und mit dem gegenüberliegenden Zahn kontaktieren. Dadurch wird das der Versiegelung gegenüberliegenden Zahnmaterials vor den abrasiven Eigenschaften der harten phosphoreszierenden Stoffe, wie sie beispielsweise für das Luminova^{®}-Produkt bekannt sind, geschont.

Das erfindungsgemäße Dentalmaterial als organische Bindemittel kann insbesondere Polymerisierbare Acrylsäureester und/oder Methacrylsäureester (ggf. auch entsprechende Oligomere und/oder Präpolymere) enthalten. Weitere geeignete organische Bindemittel insbesondere Bindemittel ohne freie Säuregruppe sind in der WO 2005/084611 genannt.

Ein erfindungsgemäßer Fissurenversiegler kann als organische Bindemittel insbesondere Bis-GMA oder Urethandimethacrylat (UDMA) enthalten. Da diese eine relativ hohe Viskosität aufweisen, können niedriger viskose polymerisierbare Stoffe wie beispielsweise Methylmethacrylat (MMA), Triethylenglycoldimethacrylat (TEDMA), Tetraethylenglycoldimethacrylat (TEGDMA), Hydroxyethylmethacrylat (HEMA)oder Glycerindimethacrylat (GDMA) hinzugefügt werden.

Zur Erhöhung der Abriebfestigkeit können neben Glaspulver weitere anorganische Füllstoffe beispielsweise auf der Basis von Siliziumdioxid, Glaskeramikpulver, Pulver von Zementen wie beispielsweise Ionomerzementen oder dergleichen hinzugegeben werden. Das Dentalmaterial enthält erfindungsgemäß fluoridhaltige Gläser.

Die Füllstoffe können auf bekannte Weise oberflächenbehandelt, z. B. silanisiert sein.

Bei einem erfindungsgemäßen Fissurenversiegler kann nach dem Auftragen und bei Bedarf in regelmäßigen Zeitabständen eine Kontrolle der ordnungsgemäßen Versiegelung durch Bestrahlung mit einer Lichtquelle und anschließender Inaugenscheinnahme im Dunkeln durchgeführt werden. Ferner hat ein erfindungsgemäßer Fissurenversiegler einen Nachleuchteffekt im Dunkeln (glow in the dark effect), der insbesondere bei Kindern und Jugendlichen als modisches Accessoire die Akzeptanz der Fissurenversiegelung erhöhen kann.

Das erfindungsgemäße Dentalmaterial kann ein Einkomponentensystem sein, insbesondere ein lichthärtendes Einkomponentensystem. Geeignete Fotoinitiatoren beispielsweise auf der Basis von Campherchinon sind dem Fachmann geläufig.

Die Erfindung wird nachfolgend ohne Einschränkung der Allgemeinheit anhand von Ausführungsbeispielen erläutert.

### Beispiel 1: Charakterisierung des in dem Fissurenversieglern gemäß Beispiel 2 verwendeten Grundharzes

Als Grundharz für die Herstellung der Fissurenversiegler gemäß Beispiel 2 wurde eine Harzmischung mit folgenden Komponenten hergestellt:
20,00 Gew.-% Bisphenol-A-diglycidylmethacrylat (BisGMA),
45,00 Gew.-% Triethylenglycoldimethacrylat (TEDMA),
33,89 Gew.-% Uretandimethacrylat (UDMA),
0,01 Gew.-% 2,6-Di-t-butyl-1-hydroxytoluol (BHT),
0,40 Gew.-% Campherchinon und
0,70 Gew.-% 2-(Ethylhexyl)-4-(Dimethylamino)Benzoate.

### Beispiel 2: Herstellung der Fissurenversieglerpasten und Prüfkörper und Untersuchung ihrer Eigenschaften

Es wurden die nachfolgenden unterschiedlichen Fissurenversieglerpasten mit folgender Zusammensetzung:
59 Gew.-% Harz gemäß Beispiel 1,
5 Gew.-% Aerosil R7200,
1 Gew.-% phosphoreszierender Stoff und
35 Gew.-% Fluoridfreisetzendes Dentalglas der Zusammensetzung:
   30 Mass.% SiO₂
   20 Mass.% Al₂O₃
   20 Mass.% SrO
   20 Mass.% F
   < 10 Mass.% ZnO
   < 5 Mass.% P₂O₅
   < 5 Mass.% Na₂O
wobei
- Paste 1:: Lumilux^{®} Effekt Grün N-CO (Zinksulfid dotiert mit Kupfer der Fa. Honeywell als phosphoreszierenden Stoff),
- Paste 2:: Super-LumiNova GL hellgrün (Strontiumoxidaluminat) als phosphoreszierenden Stoff und,
- Paste 3:: Super-LumiNova GL hellblau (Strontiumoxidaluminat) als phosphoreszierenden Stoff enthält,
in folgender Art und Weise hergestellt:
Zunächst wird in das Harz Aerosil und das Phosphoreszenzpigment eindispergiert. Anschließend wird das ionenfreisetzende Dentalglas portionsweise eingearbeitet. Die erhaltene Paste wird homogenisiert und anschließend im Vakuum entgast.
Aus den Pasten wurden durch Bestrahlung mit UV-Licht 1 mm dicke kreisförmige Prüfkörper mit einem Durchmesser von ca. 3 cm erstellt.

### Phosphoreszenzeigenschaften:

Nach Anregung durch weißes Tageslicht oder UV-Licht (254 nm) beginnen die Prüfkörper neongrün zu leuchten. Das Leuchten hält bis zu 2 Stunden an und ist besonders intensiv direkt nach Bestrahlung mit einer UV-Lampe.

### Fluoridfreisetzung:

Die Fluoridfreisetzung wurde bestimmt gemäß der Methode nach: El Mallakh, B.F., Sarkar, N.K.: Fluoride release from glass-ionomer cements in deionized water and artificial saliva. Dent Mater 6, 118-122 (1990).

Der aus der Paste 1 hergestellte Prüfkörper weist nach 28 Tagen kumulativ eine Floridfreisetzung von 13,5±2,2 ppm auf.

## Patentansprüche

1. Durch Polymerisation aushärtbares Dentalmaterial, **dadurch gekennzeichnet, dass** es einen phosphoreszierenden Stoff und fluoridfreisetzendes Dentalglas enthält.

2. Dentalmaterial nach Anspruch 1, **dadurch gekennzeichnet, dass** es ein Fissurenversiegler ist.

3. Dentalmaterial nach Anspruch 1 oder 2, **dadurch gekennzeichnet, dass** es Acrylsäureester und/oder Methacrylsäureester enthält.

4. Dentalmaterial nach einem der Ansprüche 1 bis 3, **dadurch gekennzeichnet, dass** der phosphoreszierende Stoff Sulfide von Erdalkalimetallen und/oder Zink umfasst.

5. Dentalmaterial nach einem der Ansprüche 1 bis 3, **dadurch gekennzeichnet, dass** der phosphoreszierende Stoff Erdalkalioxidaluminate umfasst.

6. Dentalmaterial nach Anspruch 5, **dadurch gekennzeichnet, dass** der phosphoreszierende Stoff Strontiumoxidaluminate umfasst.

7. Dentalmaterial nach einem der Ansprüche 1 bis 6, **dadurch gekennzeichnet, dass** es neben dem Dentalglas zusätzliche anorganische Füllstoffe umfasst.

8. Dentalmaterial nach einem der vorangehenden Ansprüche, **dadurch gekennzeichnet, dass** der Füllstoffgehalt am Dentalmaterial bis zu 70 Gew.-%, vorzugsweise bis zu 60 Gew.-%, weiter vorzugsweise bis zu 50 Gew.-%, weiter vorzugsweise bis zu 40 Gew.-%, besonders bevorzugt 30-40 Gew.-% beträgt.

9. Dentalmaterial nach einem der Ansprüche 1 bis 8, **dadurch gekennzeichnet, dass** es ein Einkomponentensystem ist.

10. Dentalmaterial nach einem der Ansprüche 1 bis 9, **dadurch gekennzeichnet, dass** es lichthärtend ist.

11. Dentalmaterial nach einem der vorangehenden Ansprüche, **dadurch gekennzeichnet, dass** das Dentalglas
10-50 Gew.-% F,
1-10 Gew.-% Na₂O
10-50 Gew.-% Al₂O₃,
10-50 Gew.-% SiO₂,
1-10 Gew.-% P₂O₅,
1-10 Gew.-% ZnO und
10-50 Gew.-% SrO enthält.

12. Dentalmaterial nach einem der vorangehenden Ansprüche, **dadurch gekennzeichnet, dass** das Dentalglas eine durchschnittliche Teilchengröße kleiner 10 *µ*m, bevorzugt kleiner 5 *µ*m, weiter bevorzugt kleiner 3 *µ*m, weiter bevorzugt kleiner 2 *µ*m, weiter bevorzugt kleiner 1 *µ*m, besonders bevorzugt kleiner 0,5 *µ*m aufweist.

13. Dentalmaterial nach einem der vorangehenden Ansprüche, **dadurch gekennzeichnet, dass** die maximale Teilchengröße des Dentalglases kleiner 40 *µ*m, weiter bevorzugt kleiner 30 *µ*m, weiter bevorzugt kleiner 20 *µ*m, weiter bevorzugt kleiner 10 *µ*m, weiter bevorzugt kleiner 5 *µ*m, weiter bevorzugt kleiner 4 *µ*m, weiter bevorzugt kleiner 3 *µ*m, besonders bevorzugt kleiner 2 *µ*m ist.

14. Dentalmaterial nach einem der vorangehenden Ansprüche, **dadurch gekennzeichnet, dass** der phosphoreszierende Stoff eine durchschnittliche Teilchengröße kleiner 10 *µ*m, bevorzugt kleiner 5 *µ*m, weiter bevorzugt kleiner 3 *µ*m, weiter bevorzugt kleiner 2 *µ*m, weiter bevorzugt kleiner 1 *µ*m, besonders bevorzugt kleiner 0,5 *µ*m aufweist.

15. Dentalmaterial nach einem der vorangehenden Ansprüche, **dadurch gekennzeichnet, dass** die maximale Teilchengröße des phosphoreszierenden Stoffes kleiner 40 *µ*m, weiter bevorzugt kleiner 30 *µ*m, weiter bevorzugt kleiner 20 *µ*m, weiter bevorzugt kleiner 10 *µ*m, weiter bevorzugt kleiner 5 *µ*m, weiter bevorzugt kleiner 4 *µ*m, weiter bevorzugt kleiner 3 *µ*m, besonders bevorzugt kleiner 2 *µ*m ist.

16. Dentalmaterial nach einem der Ansprüche 1 bis 15, **dadurch gekennzeichnet, dass** das fluoridfreisetzende Dentalglas ein Ionomerglas ist.
